# EUROPEAN PATENT SPECIFICATION

(11) **EP 1 663 129 B1**
(45) Date of publication and mention of the grant of the patent: **02.07.2008**
(21) Application number: 04764583.3
(22) Date of filing: 27.08.2004
(51) Int. Cl.: A61K 8/18, A61K 8/99

(54) **HAIR CARE COMPOSITION**
HAARBEHANDLUNGSZUSAMMENSETZUNG
COMPOSITION POUR LE SOIN DES CHEVEUX

(30) Priority: 17.09.2003 EP 03255820
(43) Date of publication of application: 07.06.2006
(73) Proprietor: UNILEVER PLC, London EC4P 4BQ (GB); UNILEVER N.V., 3013 AL Rotterdam (NL)
(72) Inventor: PRATLEY, Stuart K., Unilever R & D Port Sunlight, Bebington, Wirral Merseyside CH63 3JW (GB); VAN-VUURE, Aart, Randwijk 6668AW (NL)
(74) Representative: Tansley, Sally Elizabeth
(86) International application number: PCT/EP2004/009609
(87) International publication number: WO 2005/025525

(56) References cited:
- EP-A- 0 784 972
- WO-A-01/17502
- US-A1- 2003 161 796

## Description

### Field of the Invention

This invention relates to hair care compositions, in particular to hair care compositions that style hair.

### Background and Prior Art

The desire to have the hair retain a particular shape or style is widely held. Styling compositions are usually applied in the form of, sprays, mousses, gels and lotions. However, in some countries creams are a particularly popular way of styling hair. A major disadvantage with using styling creams is that they have a tendency to feel sticky both in the pot before styling and on the hair after styling.

US2003/161796 describes a hair treatment composition, e.g. a cream comprising an aqueous phase comprising a surfactant, a fatty acid and a liquid emollient, such as silicones or glycerides.

WO0117502 refers to a hair care composition such as a cream comprising an aqueous phase comprising a cationic surfactant, a solid fatty acid and a liquid emollient.

EP0784972 describes cosmetic and/or pharmaceutical emulsions comprising a) alkyl oligoglucosides b) fatty alcohols and/or c) polyolpoly-12-hydroxy stearates and d) Guerbet alcohols.

The present invention is a hair styling cream that helps mitigate the problem of stickiness.

Further advantages of the creams of the present invention are: they can be used to maintain a style; enable re-styling of hair; condition hair; reduce hair damage; provide hair shine; provide a wet look; control hair volume and align the hair.

### Description of the Present Invention

According to the present invention, there is provided a hair styling cream comprising
i) an aqueous lamellar phase comprising a) cationic surfactant and b) fatty alcohol having a melting point greater than 25°C and/or fatty acid having a melting point greater than 40°C; and
ii) 20 wt% or greater of one or more non-volatile liquid emollient(s), the viscosity of any single non-volatile emollient or the viscosity of a blend of non-volatile emollients being less than 1000 mPa.s at 25°C and 5 s⁻¹.

The invention also relates to a method of styling hair by applying to the hair a composition as described above.

Also described is the use of the above composition for styling hair.

### Detailed Description of the Invention

In the context of the present invention a cream is defined as not immediately pourable under gravity.

It is preferable if the viscosity of the final cream is from 10,000 to 300,000 mPa.s at 5 s-1 and 25°C, more preferably from 20,000 to 200,000 mPa.s, and most preferably between 30,000 and 150,000 mPa.s

The viscosity of the fluids was determined with a standard stress controlled rheometer (this case Carrimed CSL-100), using a parallel plate configuration at a gap height of 200 µm and by measuring viscosity at a range of shear rates.

### Cationic Surfactant

Suitable cationic surfactants comprise one or more conditioning surfactants, which are cosmetically acceptable and suitable for topical application to the hair.

Cationic surfactants useful in compositions of the invention contain amino or quaternary ammonium hydrophilic moieties which are positively charged when dissolved in the aqueous composition of the present invention.

Examples of suitable cationic surfactants are those corresponding to the general formula:

[N(R₁)(R₂)(R₃)(R₄)]⁺ (X)⁻

in which R₁, R₂, R₃, and R₄ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

Examples of suitable cationic surfactants include: quaternary ammonium chlorides, e.g. alkyltrimethylammonium chlorides wherein the alkyl group has from about 8 to 22 carbon atoms, for example octyltrimethylammonium chloride, dodecyltrimethylammonium chloride, stearyl trimethyl ammonium chloride, behenyl trimethyl ammonium chloride, hexadecyltrimethyl-ammonium chloride, cetyltrimethylammonium chloride, octyldimethylbenzylammonium chloride, decyldimethylbenzyl-ammonium chloride, stearyldimethylbenzylammonium chloride, didodecyldimethylammonium chloride, dioctadecyldimethylammonium chloride, tallow trimethylammonium chloride, cocotrimethylammonium chloride, and the corresponding salts thereof, e.g., bromides, hydroxides. Cetylpyridinium chloride or salts thereof, e.g., chlorideQuaternium -5, Quaternium -31, Quaternium -18 and mixtures thereof.

Preferred are stearyl trimethyl ammonium chloride, hardened tallow trimethyl ammonium chloride, particularly preferred cationic surfactants are cetrimonium chloride, behenyl trimethyl ammonium chloride, di hardened-tallow dimethyl ammonium chloride, distearyl dimethyl ammonium chloride, N,N-bis(2-hydroxyethyl)methyl octadecenyl ammonium chloride or mixtures thereof.

In the conditioners of the invention, the level of cationic surfactant is preferably from 0.05 to 12, more preferably 0.1 to 8, most preferably 0.2 to 5 wt% of the total composition.

### Fatty acid/Fatty alcohol

The composition has as an essential feature of the invention a fatty alcohol having a melting point greater than 35°C and/or a fatty acid having a melting point greater than 40 °C, more preferably the fatty acid has a melting point greater than 50°C.

The level of fatty alcohol and/or fatty acid within phase i) of the composition is from 0.2 wt% to 15 wt% of the total composition, more preferably from 0.3 wt% to 10 wt%.

The fatty alcohol is preferably selected from the group consisting of cetyl alcohol, stearyl alcohol, behenyl alcohol or mixtures thereof.

The fatty acid is preferably a C16 to C22 acid. Examples of suitable fatty acids include palmitic acid and stearic acid.

It is preferable if the ratio of fatty acid and or fatty alcohol(i.e. component b) to total cationic surfactant (component a) within the lamellar phase is from 0.5 to 10.0, preferably from 1 to 10.0, and most preferably from 1.0 to 7.0.

### Non-Volatile Liquid Emollients

A non-volatile emollient is defined such that when the emollient is placed in a petri-dish in a room at standard environmental conditions (25°C, 50%RH) at a fluid height of 3 mm, after 1 hour less than 10 wt% of the emollient will have evaporated.

In the present invention the viscosity of the non-volatile liquid emollient refers to a single emollient or to the total viscosity of a blend of emollients.

The viscosity of each of the non-volatile liquid emollients is preferably less than 200 mPa.s and most preferably less than 50 mPa.s at 25°C and 5s⁻¹. If a blend of emollients is used a high viscosity emollient can be diluted with a low viscosity emollient to bring the average viscosity of the blend to less than 200 mPa.s, preferably less than 50 mPa.s.

In some cases lipophilic emollients are preferred. Preferred lipophilic emollients are selected from the group consisting of triglycerides, fatty esters, mineral oils (branched hydrocarbons) and mixtures thereof. Preferred triglycerides include triheptanoin, tricaprylin, tricaprin, triundecanoin, trilinolein, triolein, almond oil, coconut oil, olive oil, palm kernel oil, peanut oil or sunflower oil. Preferred fatty esters include isocetyl stearate. Preferred mineral oils have an average hydrocarbon chain length above 20 carbon units. Another preferred oil is dicaprylate/dicaprate propylene glycol. Mixtures of the above emollients may be used.

Suitable mineral oils are those sold under the name Sirius White Oils by Fuchs Lubricants (UK). Examples of suitable oils are Sirius M85, Sirius M125 and Sirius M350.

The emollient may also be a silicone oil with viscosity as previously defined, preferably dimethicone.

The emollient ii) may also be hydrophilic, if hydrophilic it is preferably selected from the group consisting of polyethylene glycol with a molecular weight preferably from 250 g/mol to 700 g/mol, or polypropylene glycol with a molecular weight preferably from 350 g/mol to 2000 g/mol.

The level of emollient(s) ii) within the total hair cream composition is preferably from 20 wt% to 70 wt%, more preferably from 30 wt% to 50 wt% of the total composition.

One or all of the emollient components can be included as a pre-emulsion.

### Styling Compound

In some aspects of this invention it is desirable if the composition comprises an additional styling aid.

Particularly useful as styling aids with this invention are hair styling polymers. Hair styling polymers are well known articles of commerce and many such polymers are available commercially which contain moieties, which render the polymers cationic, anionic, amphoteric or nonionic in nature. The polymers may be synthetic or naturally derived. Cationic and nonionic polymers are preferred, however nonionic polymers are particularly preferred.

The amount of the hair styling polymer may range from 0.1 to 10%, preferably 0.5 to 8 %, more preferably 0.75 to 6% by weight based on total weight of the composition.

Examples of nonionic hair styling polymers are homopolymers of N- vinylpyrrolidone and copolymers of N-vinylpyrrolidone with compatible nonionic monomers such as vinyl acetate. Nonionic polymers containing N- vinylpyrrolidone in various weight average molecular weights are available commercially from ISP Corporation - specific examples of such materials are homopolymers of N-vinylpyrrolidone having an average molecular weight of about 630,000 sold under the name PVP K-90 and homopolymers of N-vinylpyrrolidone having an average molecular weight of about 1,000,000 sold under the name of PVP K-120. Particularly preferred is a copolymer of polyvinyl pyrrolidone and polyvinyl acetate. An example of this copolymer is sold by BASF under the name Luviskol VA64.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as N-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic hair styling polymers are:
copolymers of N-vinylpyrrolidone and dimethylaminoethyl methacrylate, available from ISP Corporation as Copolymer 845, Copolymer 937 and Copolymer 958;
copolymers of N-vinylpyrrolidone and dimethylaminopropylacrylamide or methacrylamide, available from ISP Corporation as Styleze® CC10;
copolymers of N-vinylpyrrolidine and dimethylaminoethyl methacrylate;
copolymers of vinylcaprolactam, N-vinylpyrrolidone and dimethylaminoethylmethacrylate;
Polyquaternium-4 (a copolymer of diallyldimonium chloride and hydroxyethylcellulose);
Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Luviquat® PQ11;
Polyquaternium-16 (formed from methylvinylimidazolium chloride and vinylpyrrolidone), available from BASF as Luviquat® FC 370, FC 550, FC 905 and HM-552;
Polyquaternium-46 (prepared by the reaction of vinylcaprolactam and vinylpyrrolidone with methylvinylimidazolium methosulphate), available from BASF as Luviquat®Hold.

Examples of suitable naturally-derived hair styling polymers include shellac, alginates, gelatins, pectins, cellulose derivatives and chitosan or salts and derivatives thereof. Commercially available examples include Kytamer® (ex Amerchol) and Amaze® (ex National Starch).

### Further Components

Such styling products frequently include a carrier and further additional components. The carriers and additional components required to formulate such products vary with product type and can be routinely chosen by one skilled in the art. The following is a description of some of these carriers and additional components.

An aqueous phase thickener is preferably present and can be based on a cellulose derivative, in particular hydroxyethyl cellulose or cetyl hydroxyethyl cellulose. Such aqueous phase thickeners are typically present in an amount from 0.01% to 10% by weight.

Hair care compositions of the present invention can comprise a carrier, or a mixture of such carriers, which are suitable for application to the hair. The carriers are present at from about 0.5% to about 99.5%, preferably from about 5.0% to about 99.5%, more preferably from about 10.0% to about 98.0%, of the composition. As used herein, the phrase "suitable for application to hair" means that the carrier does not damage or negatively affect the aesthetics of hair or cause irritation to the underlying skin.

Compositions according to the invention comprise a buffer or pH adjuster. Preferred buffers or pH adjusters include weak acids and bases such as glycine/sodium hydroxide, citric acid, triethanolamine, lactic acid, succinic acid, acetic acid and salts thereof. Frequently a mixture of buffering system is used such as sodium citrate and citric acid.

Carriers suitable for use with hair care compositions of the present invention include, for example, those commonly used in creams. The carriers used herein can include a wide range of components conventionally used in hair care compositions. The carriers can contain a solvent to dissolve or disperse the styling compound being used, with water, the C₁-C₆ alcohols, lower alkyl acetate and mixtures thereof being preferred. The carriers can also contain a wide variety of additional materials such as acetone, hydrocarbons (such as isobutane, hexane, decene), water, ethanol, volatile silicone derivatives, and mixtures thereof. The solvents used in such mixtures may be miscible or immiscible with each other.

The carrier can include a wide variety of further conditioning materials suitable for hair such as quaternary silicone polymers, silicone based conditioners and their emulsions, and amino functional silicones and their emulsions. The viscosity of these conditioning silicones is greater than 10,000 mPa.s at 25°C and 5 s⁻¹.

Further general ingredients suitable for all product forms include, sun-screening agents, preservatives, anti-oxidants, anti-dandruff actives, and emulsifiers for emulsifying the various carrier components of the compositions of the invention.

The compositions of the present invention may also contain adjuncts suitable for hair care. Generally such ingredients are included individually at a level of up to 2, preferably up to 1 wt% of the total composition. Suitable hair care adjuncts, include amino acids, sugars and ceramides.

Compositions of the present invention are formulated into hair care compositions, especially products with hair styling claims. The compositions are for use in styling human hair and, more preferably, they are packaged and labelled as such.

It is preferred if the products are left on hair after application and not immediately washed off.

The following non-limiting Examples further illustrate the preferred embodiments of the invention. All percentages referred to in the examples and throughout this specification are by weight based on total weight unless otherwise indicated.

Examples of the invention are illustrated by a number, comparative examples are illustrated by a letter.

### EXAMPLES

### Example 1, Example 2 and Example A

| Required levels of oil | | | | |
|---|---|---|---|---|
| Trade Name | Chemical name | % active ingredient | | |
| | | Example 1 | Example 2 | Example A |
| Trivent OCG | Tricaprylin | 30 | 20 | 0 |
| Arquad 1650 | Cetrimoniumchloride + isopropylalcohol | 0.7 + 0.7 | 0.7+0.7 | 0.7+0.7 |
| Laurex CS | Cetyl/Stearyl alcohol | 4.2 | 4.2 | 4.2 |
| DC 2-1784 HVF | Silicone emulsion | 2.1 | 2.1 | 2.1 |
| Polysurf 67 | Hydroxyethylcellulo se | 0.1 | 0.1 | 0.1 |
| Nipagin M | Methyl paraben | 0.2 | 0.2 | 0.2 |
| Additin RC 7110 | BHT | 0.05 | 0.05 | 0.05 |
| Nipasol M | Propyl paraben | 0.1 | 0.1 | 0.1 |
| Sepicide LD | Phenoxyethanol | 0.4 | 0.4 | 0.4 |
| TEA | Triethanolamine | 0.7 | 0.7 | 0.7 |
| Perfume | Perfume | 0.1 | 0.1 | 0.1 |
| Water | Distilled water | to 100 | to 100 | to 100 |

The lamellar phase creams were prepared by mixing the methyl paraben, BHT, CTAC and cetearyl alcohol in 70% of the total water at 75°C under low shear and then under vacuum at high shear. A hydroxyethyl cellulose dispersion was prepared in the remaining 30% of the water and heated to 75°C under low shear. The cellulose dispersion was then added to the initial mixture under low shear, followed by addition of the oil (tricaprylin). 5 minutes of high shear under vacuum was then undertaken. The resultant mixture was slowly cooled with low shear mixing. Silicone fluid and phenoxy ethanol were then added. Triethanolamine was added , followed by perfume and then high shear mixing under vacuum was conducted.

The Examples were applied to hair switches and the styling performance was assessed. For this purpose 0.5g of hair cream was applied to a straight, flat, 2.5 cm wide, 25 cm long hair switch weighing 5g. The cream was spread through the hair and subsequently the hair was pressed together to create a volume down effect. After styling the frontal area of each switch was assessed by image analysis. Performance was benchmarked against a current commercial hair cream material (referred to as benchmark). The following results were obtained:

| Material no. | Concentration oil: | Switch frontal area (mm2): |
|---|---|---|
| Benchmark | | 5900±400 |
| 1 | 30 | 5500±400 |
| 2 | 20 | 6500±400 |
| A | 0 | 7500±400 |

These results show that to achieve parity performance with the benchmark at least 20% of oil needs to be used in the composition.

### Example 1 vs Example B

The performance of Example 1 above (CTAC lamellar gel phase + 30% tricaprylin) was compared to comparative Example B. Example B did not have the required lamellar phase.

Tricaprylin has a dynamic viscosity of 20 mPas at 25°C and 5s⁻¹ Laurex CS has a melting point of 50-55°C.

### Example B

| Trade Name | Chemical name | % active ingredient |
|---|---|---|
| Trivent OCG | Tricaprylin | 30 |
| Tween 60 | Polyoxyethylene sorbitan monostearate (20EO) | 7.6 |
| Span 60 | Sorbitan monostearate | 1.9 |
| Nipagin M | Methyl paraben | 0.2 |
| Nipasol M | Propyl paraben | 0.1 |
| BHT | Dibutylhydroxytoluene | 0.05 |
| Sepicide LD | Phenoxyethanol | 0.4 |
| Carbopol 940 | Carboxyvinyl polymer | 0.3 |
| Perfume | Perfume | 0.1 |
| Water | Distilled water | to 100 |

Example B was prepared as a normal Oil-in-Water emulsion.

Examples 1 and B were evaluated by a trained quantitative consumer panel and the following results were obtained (attributes scored on a scale from 0 to 100):

| Attribute | Example 1 | Example B |
|---|---|---|
| Stickiness of hair | 32 | 39 |
| Ease of styling | 66 | 60 |
| Hold of style | 61 | 58 |

Examples 1 and B were further evaluated by a panel (80 people) and the following results were obtained (attributes scored on a scale from 0 to 5):

| Attribute | Example 1 | Example B |
|---|---|---|
| Can create style | 3.11 | 2.84 |
| Hair is not sticky | 3.73 | 3.57 |
| Initial hold of style | 3.11 | 2.82 |
| Hold of style during the day | 3.08 | 2.79 |

Based on these results it was concluded that the lamellar gel cream + oil gives a better balance of styling properties and good sensory feel than the non-lamellar phase cream base + oil.

The following are also examples of stable formulations according to the invention, which give good styling properties to hair without feeling sticky on application.

### Examples with differing cationic surfactants

| | | | | | | |
|---|---|---|---|---|---|---|
| Trade Name | Chemical name | % active ingredient/**Example** | | | | |
| | | **3** | **4** | **5** | **6** | **7** |
| Trivent OCG | Tricaprylin | 20 | 30 | 30 | 30 | 30 |
| Arquad 1650 | Cetrimoniumchl oride (50%) + isopropylalcoh ol | 0.7 + 0.7 | | | | |
| Genamin KDMP | Behenyl Trimethyl Ammonium Chloride (80%) + isopropanol | | 2.0 + 0.5 | 4.0 + 1.0 | | |
| Ethoquad O/12 PG | PEG-2 Oleammonium chloride (75%) + propylene | | | | | 1.99 |
| | glycol | | | | | 0.66 |
| Genamin CTAC | Cetrimonium chloride (29% solution) | | | | 1.6 | |
| Arquad 2HT-75 PG | Ditallowdimeth ylammonium chloride (75%) + propylene glycol | | | | 0.8+0.27 | |
| Laurex CS | Cetyl/Stearyl alcohol | | 4.0 | 8.0 | 5.0 | 7.0 |
| Pristerine 4900 | Stearic acid | 4.2 | | | | |
| DC 2-1784 HVF | Silicone emulsion | 2.1 | 2.1 | 2.1 | 2.1 | 2.1 |
| Polysurf 67 | Hydroxyethylce llulose | 0.1 | 0.1 | 0.1 | 0.1 | 0.1 |
| Nipagin M | Methyl paraben | 0.2 | 0.2 | 0.2 | 0.2 | 0.2 |
| Additin RC 7110 | BHT | 0.05 | 0.05 | 0.05 | 0.05 | 0.05 |
| Nipasol M | Propyl paraben | 0.1 | 0.1 | 0.1 | 0.1 | 0.1 |
| Sepicide LD | Phenoxyethanol | 0.4 | 0.4 | 0.4 | 0.4 | 0.4 |
| TEA | Triethanolamin e | 0.7 | 0.7 | 0.7 | 0.7 | 0.7 |
| Perfume | Perfume | 0.2 | 0.2 | 0.2 | 0.2 | 0.2 |
| Water | Distilled water | to 100 | to 100 | to 100 | to 100 | to 100 |

### Examples with differing types of non volatile liquid emollients

| Trade Name | Chemical name | % active ingredient/**Examples** | | | |
|---|---|---|---|---|---|
| | | **8** | **9** | **10** | **11** |
| Sirius M350 | Mineral oil | 20 | | | |
| A&E Isocetyl stearate | Isocetyl stearate | | 20 | | |
| Estol 1526 | Dicaprylate dicaprate propylene glycol | | | 20 | |
| Efaderma F | Trilinolein | | | | 20 |
| Arquad 1650 | Cetrimoniumchloride + isopropylalcohol | 0.7 + 0.7 | 0.7+0. 7 | 0.7+0. 7 | 0.7+0. 7 |
| Laurex CS | Cetyl/Stearyl alcohol | 4.2 | 4.2 | 4.2 | 4.2 |
| DC 2-1784 HVF | Silicone emulsion | 2.1 | 2.1 | 2.1 | 2.1 |
| Polysurf 67 | Hydroxyethylcellulose | 0.1 | 0.1 | 0.1 | 0.1 |
| Nipagin M | Methyl paraben | 0.2 | 0.2 | 0.2 | 0.2 |
| Additin RC 7110 | BHT | 0.05 | 0.05 | 0.05 | 0.05 |
| Nipasol M | Propyl paraben | 0.1 | 0.1 | 0.1 | 0.1 |
| Sepicide LD | Phenoxyethanol | 0.4 | 0.4 | 0.4 | 0.4 |
| TEA | Triethanolamine | 0.7 | 0.7 | 0.7 | 0.7 |
| Perfume | Perfume | 0.1 | 0.1 | 0.1 | 0.1 |
| Water | Distilled water | to 100 | to 100 | to 100 | to 100 |

| | | | | | |
|---|---|---|---|---|---|
| Sirius M350 mineral oil has a viscosity of 80mPas (25°C, 5s⁻¹) Icocetyl stearate has a viscosity of 23mPas (25°C, 5s⁻¹) Estol 1526 has a viscosity of 9mPas (25°C, 5s⁻¹) Efaderma F has a viscosity of 54mPas (25°C, 5s⁻¹) Stearic acid has a melting point of 70°C | | | | | |

### Examples with silicone oils:

| Trade Name | Chemical name | % active ingredient/ **Examples** | |
|---|---|---|---|
| | | **12** | **13** |
| DC 200 100cS | Dimethicone | 20 | |
| DC 200 10cS | Dimethicone | | 20 |
| Arquad 1650 | Cetrimoniumchloride + isopropylalcohol | 0.7 + 0.7 | 0.7+0.7 |
| Laurex CS | Cetyl/Stearyl alcohol | 4.2 | 4.2 |
| DC 2-1784 HVF | Silicone emulsion | 2.1 | 2.1 |
| Polysurf 67 | Hydroxyethylcellulose | 0.1 | 0.1 |
| Nipagin M | Methyl paraben | 0.2 | 0.2 |
| Additin RC 7110 | BHT | 0.05 | 0.05 |
| Nipasol M | Propyl paraben | 0.1 | 0.1 |
| Sepicide LD | Phenoxyethanol | 0.4 | 0.4 |
| TEA | Triethanolamine | 0.7 | 0.7 |
| Perfume | Perfume | 0.1 | 0.1 |
| Water | Distilled water | to 100 | to 100 |

### Prototypes with humectant oils:

| Trade Name | Chemical name | % active ingredient / **Examples** | |
|---|---|---|---|
| | | **14** | **15** |
| Polyglyco 1 E-400 | PEG-8 | 20 | |
| Polyglyco 1 P400E | Polypropylene glycol Mw 400 | | 40 |
| Arquad 1650 | Cetrimoniumchloride + isopropylalcohol | 0.7 + 0.7 | 0.7+0.7 |
| Laurex CS | Cetyl/Stearyl alcohol | 4.2 | 4.2 |
| DC 2-1784 HVF | Silicone emulsion | 2.1 | 2.1 |
| Polysurf 67 | Hydroxyethylcellulose | 0.1 | 0.1 |
| Nipagin M | Methyl paraben | 0.2 | 0.2 |
| Additin RC 7110 | BHT | 0.05 | 0.05 |
| Nipasol M | Propyl paraben | 0.1 | 0.1 |
| Sepicide LD | Phenoxyethanol | 0.4 | 0.4 |
| TEA | Triethanolamine | 0.7 | 0.7 |
| Perfume | Perfume | 0.1 | 0.1 |
| Water | Distilled water | to 100 | to 100 |

| | | | |
|---|---|---|---|
| PEG-8 has a viscosity of 100 mPas (25°C, 5s⁻¹) PPG 400 has a viscosity of 50 mPas (25°C, 5s⁻¹) | | | |

## Claims

1. A hair styling cream comprising:
i) an aqueous lamellar phase comprising a) cationic surfactant and b) fatty alcohol having a melting point greater than 35°C and/or fatty acid having a melting point greater than 40 °C; and
ii) 20 wt% or greater of one or more non-volatile liquid emollient(s), the viscosity of any single non-volatile emollient or the viscosity of a blend of non-volatile emollients being less than 1000 mPa.s at 25°C and 5 s⁻¹, in which the non-volatile liquid emollient is selected from c) lipophilic emolient selected from the group consisting of triglycerides, fatty esters, mineral oils and mixtures thereof or d) hydrophilic emolient selected from the group consisting of polyethylene glycol with a molecular weight from 250 g/mol to 700 g/mol or polypropylene glycol with a molecular weight from 350 g/mol to 2000 g/mol.

2. A hair cream according to claim 1 in which the viscosity of any single emollient or the viscosity of a blend of emollients ii) is less than 50 mPa.s at 25°C and 5s⁻¹.

3. A hair cream according to any preceding claim in which the level of emollient ii) is from 30 wt% to 50 wt% of the total composition.

4. A hair cream according to any preceding claim in which the level of cationic surfactant within phase i) is from 0.2 wt% to 5 wt% of the total composition.

5. A hair cream according to any preceding claim in which the cationic surfactant is selected from the group consisting of cetrimonium chloride, behenyl trimethyl ammonium chloride, di hardened-tallow dimethyl ammonium chloride, distearyl dimethyl ammonium chloride, N,N-bis(2-hydroxyethyl)methyl octadecenyl ammonium chloride or mixtures thereof.

6. A hair cream composition according to any preceding claim in which the level of fatty alcohol and/or fatty acid (b) within lamellar phase i) is from 0.3 wt% to 10 wt% of the total composition.

7. A hair cream according to any preceding-claim in which component b within the lamellar phase i) is a fatty alcohol selected from the group consisting of cetyl alcohol, stearyl alcohol, behenyl alcohol or mixtures thereof.

8. A hair cream according to any preceding claim which further comprises an aqueous phase thickener.

9. A hair cream according to claim 11 in which the aqueous phase thickener is hydroxyethyl cellulose or cetyl hydroxyethyl cellulose.

10. A hair cream according to any preceding claim, which further comprises a nonionic styling polymer.

11. A hair cream according to any preceding claim in which the viscosity of the final cream is between 30,000 and 150,000 mPa.s at 25°C and 5s⁻¹.

12. A hair cream according to any preceding claim which is a leave on product.

13. A method of treating hair comprising the step of applying to the hair a composition as defined in any of the claims above.

14. Use of a hair composition according to any one of claims 1 to 11 for styling hair.

## Patentansprüche

1. Haarstyling-Creme, umfassend:
i) eine wässrige lamellare Phase, umfassend a) kationisches Tensid und b) Fettalkohol mit einem Schmelzpunkt größer als 35°C und/oder Fettsäure mit einem Schmelzpunkt größer als 40°C; und
ii) 20 Gew.-% oder mehr von einem oder mehreren nicht-flüchtigen flüssigen Erweichungsmittel(n), wobei die Viskosität von jedem einzelnen nicht-flüchtigen Erweichungsmittel oder die Viskosität von einem Blend von nicht-flüchtigen Erweichungsmitteln weniger als 1000 mPa.s bei 25°C und 5 s⁻¹ beträgt, wobei das nicht-flüchtige flüssige Erweichungsmittel ausgewählt ist aus c) lipophilem Erweichungsmittel, ausgewählt aus der Gruppe, bestehend aus Triglyceriden, Fettestern, Mineralölen und Gemischen davon, oder d) hydrophilem Erweichungsmittel, ausgewählt aus der Gruppe, bestehend aus Polyethylenglycol mit einem Molekulargewicht von 250 g/Mol bis 700 g/Mol oder Polypropylenglycol mit einem Molekulargewicht von 350 g/Mol bis 2000 g/Mol.

2. Haarcreme nach Anspruch 1, wobei die Viskosität von jedem einzelnen Erweichungsmittel oder die Viskosität von einem Blend von Erweichungsmitteln ii) weniger als 50 mPa.s bei 25°C und 5 s⁻¹ ist.

3. Haarcreme nach einem vorangehenden Anspruch, wobei der Anteil an Erweichungsmittel ii) von 30 Gew.-% bis 50 Gew.-% der Gesamtzusammensetzung ist.

4. Haarcreme nach einem vorangehenden Anspruch, wobei der Anteil an kationischem Tensid in der Phase i) von 0,2 Gew.-% bis 5 Gew.-% der Gesamtzusammensetzung ist.

5. Haarcreme nach einem vorangehenden Anspruch, wobei das kationische Tensid aus der Gruppe, bestehend aus Cetrimoniumchlorid, Behenyltrimethylammoniumchlorid, di-gehärtetem Dimethylammoniumchlorid, Distearyldimethylammoniumchlorid, N,N-Bis(2-hydroxyethyl)methyloctadecenylammonium-chlorid oder Gemischen davon ausgewählt ist.

6. Haarcreme nach einem vorangehenden Anspruch, wobei der Anteil an Fettalkohol und/oder Fettsäure (b) innerhalb der lamellaren Phase i) von 0,3 Gew.-% bis 10 Gew.-% der Gesamtzusammensetzung ist.

7. Haarcreme nach einem vorangehenden Anspruch, wobei Komponente b innerhalb der lamellaren Phase i) einen Fettalkohol darstellt, ausgewählt aus der Gruppe, bestehend aus Cetylalkohol, Stearylalkohol, Behenylalkohol oder Gemischen davon.

8. Haarcreme nach einem vorangehenden Anspruch, die weiterhin ein Verdickungsmittel für wässrige Phasen umfasst.

9. Haarcreme nach Anspruch 11, worin das Verdickungsmittel für wässrige Phasen Hydroxyethylcellulose oder Cetylhydroxyethylcellulose ist.

10. Haarcreme nach einem vorangehenden Anspruch, die weiterhin ein nicht-ionisches Styling-Polymer umfasst.

11. Haarcreme nach einem vorangehenden Anspruch, wobei die Viskosität der fertigen Creme zwischen 30 000 und 150 000 mPa.s bei 25°C und 5 s⁻¹ liegt.

12. Haarcreme nach einem vorangehenden Anspruch, die ein Leave-on-Produkt ist.

13. Verfahren zum Behandeln von Haar, umfassend den Schritt des Auftragens auf das Haar einer Zusammensetzung wie in einem der vorstehenden Ansprüche definiert.

14. Verwendung einer Haarzusammensetzung nach einem der Ansprüche 1 bis 11 zum Styling von Haar.

## Revendications

1. Crème de coiffage capillaire comprenant
i) une phase lamellaire aqueuse comprenant a) un tensioactif cationique et b) un alcool gras ayant un point de fusion supérieur à 35 °C et/ou un acide gras ayant un point de fusion supérieur à 40 °C ; et
ii) 20 % en poids ou plus d'un ou plusieurs émollients liquides non volatils, la viscosité d'un quelconque émollient non volatil unique ou la viscosité d'un mélange d'émollients non volatils étant inférieure à 1000 mPa·s à 25 °C et 5 s⁻¹,
dans laquelle l'émollient liquide non volatil est choisi parmi c) un émollient lipophile choisi dans le groupe constitué par les triglycérides, les esters gras, les huiles minérales et leurs mélanges, ou d) un émollient hydrophile choisi dans l'ensemble constitué par le polyéthylèneglycol ayant une masse moléculaire de 250 g/mol à 700 g/mol et le polypropylèneglycol ayant une masse moléculaire de 350 g/mol à 2000 g/mol.

2. Crème capillaire selon la revendication 1, dans laquelle la viscosité d'un quelconque émollient unique ou la viscosité d'un mélange d'émollients ii) est inférieure à 50 mPa·s à 25 °C et 5 s⁻¹.

3. Crème capillaire selon l'une quelconque des revendications précédentes, dans laquelle le taux d'émollient ii) est de 30 % en poids à 50 % en poids de la composition totale.

4. Crème capillaire selon l'une quelconque des revendications précédentes, dans laquelle le taux de tensioactif cationique dans la phase i) est de 0,2 % en poids à 5 % en poids de la composition totale.

5. Crème capillaire selon l'une quelconque des revendications précédentes, dans laquelle le tensioactif cationique est choisi dans l'ensemble constitué par le chlorure de cétrimonium, le chlorure de béhényl triméthyl ammonium, le chlorure de di(suifyl hydrogéné)diméthylammonium, le chlorure de distéaryldiméthylammonium, le chlorure de N,N-bis(2-hydroxyéthyl)méthyloctadécénylammonium et leurs mélanges.

6. Composition de crème capillaire selon l'une quelconque des revendications précédentes, dans laquelle le taux d'alcool gras et/ou d'acide gras (b) dans la phase lamellaire i) est de 0,3 % en poids à 10 % en poids de la composition totale.

7. Crème capillaire selon l'une quelconque des revendications précédentes, dans laquelle le composant b dans la phase lamellaire i) est un alcool gras choisi dans l'ensemble constitué par l'alcool cétylique, l'alcool stéarylique, l'alcool béhénylique et leurs mélanges.

8. Crème capillaire selon l'une quelconque des revendications précédentes, qui comprend en outre un épaississant de phase aqueuse.

9. Crème capillaire selon la revendication 11, dans laquelle l'épaississant de phase aqueuse est l'hydroxyéthylcellulose ou la cétylhydroxyéthylcellulose.

10. Crème capillaire selon l'une quelconque des revendications précédentes, qui comprend en outre un polymère coiffant non-ionique.

11. Crème capillaire selon l'une quelconque des revendications précédentes, dans laquelle la viscosité de la crème finale est comprise entre 30 000 et 150 000 mPa·s à 25 °C et 5 s⁻¹.

12. Crème capillaire selon l'une quelconque des revendications précédentes, qui est un produit ne se rinçant pas.

13. Procédé pour traiter les cheveux, comprenant l'étape d'application aux cheveux d'une composition telle que définie dans l'une quelconque des revendications ci-dessus.

14. Utilisation d'une composition capillaire selon l'une quelconque des revendications 1 à 11 pour coiffer les cheveux.
